# EUROPEAN PATENT APPLICATION

(11) **EP 3 903 775 A1**
(43) Date of publication of application: **03.11.2021**
(21) Application number: 19906114.4
(22) Date of filing: 27.12.2019
(51) Int. Cl.: A61K 31/135, A61P 25/00, A61P 25/18, A61P 25/28

(54) **R-KETAMINE AND DERIVATIVE THEREOF AS PROPHYLACTIC OR THERAPEUTIC AGENT FOR NEURODEVELOPMENTAL DISORDER**

(30) Priority: 27.12.2018 JP 2018245688
(71) Applicant: National University Corporation Chiba University, Chiba-shi, Chiba 263-8522 (JP)
(72) Inventor: HASHIMOTO, Kenji, Chiba-shi, Chiba 260-8670 (JP)
(74) Representative: Cooley (UK) LLP
(86) International application number: PCT/JP2019/051605
(87) International publication number: WO 2020/138491

(57) **Abstract**

The present invention provides a preventive or therapeutic agent for a neurodevelopmental disorder that includes the compound illustrated in formula (I) below, or a pharmaceutically acceptable salt thereof, as an active ingredient.
Formula (I):

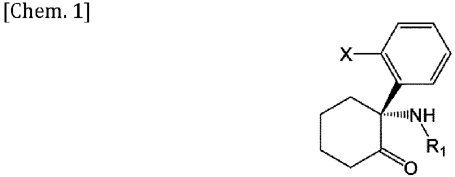

## Description

### [Technical Field]

The present invention relates to a preventive or therapeutic agent for a neurodevelopmental disorder including R-ketamine and a derivative thereof.

### [Background Art]

As a neurodevelopmental disorder, schizophrenia is a representative mental illness that occurs at a rate of about 1% of the population regardless of race or region, often appearing from puberty to adolescence. In Japan, patients hospitalized for schizophrenia occupy about 15% of the total number of hospital beds, which is a major problem in terms of medical economics as well. As symptoms, there are positive symptoms such as hallucination and delusion; negative symptoms such as blunted or flat affect, diminished volition, reduced thinking, and social withdrawal; and cognitive impairments such as reduced attention, reduced working memory, and a reduced executive function. Among these symptoms, cognitive impairments in particular have a possibility of being core symptoms of schizophrenia; they are thought to lower the quality of life (QOL) of patients and are an obstacle to social rehabilitation for patients (non-patent literatures 1, 2).

Pharmacotherapy is essential in treating schizophrenia, and antipsychotics such as phenothiazine compounds, butyrophenone compounds, benzamide compounds, iminodibenzyl compounds, thiepine compounds, indole compounds, and serotonin and dopamine receptor antagonists are in use. Antipsychotics in actual clinical use are effective against positive symptoms such as hallucination and delusion but are hardly effective against negative symptoms and cognitive impairments. As such, the development of a therapeutic drug for negative symptoms and cognitive impairments is in high demand in Japan and abroad.

Administering phencyclidine (phencyclidine; also "PCP" hereinbelow), which is known to be an NMDA receptor antagonist, to a healthy individual causes symptoms very similar to schizophrenia (positive symptoms, negative symptoms, cognitive impairments) (non-patent literatures 3, 4). As such, PCP is widely used to create animal models of schizophrenia (non-patent literature 5). In particular, a system using a novel object recognition test (NORT: novel object recognition test) with mice repeatedly administered with PCP is reportedly useful as animal models of the negative symptoms and cognitive impairments of schizophrenia (non-patent literatures 6 to 8).

Furthermore, RS-ketamine, an NMDA receptor antagonist, is also administered to healthy individuals and animals as models of schizophrenia (non-patent literatures 9, 10).

RS-ketamine is used as an anesthetic, but side effects such as psychotic symptoms, such as hallucination and delusion; dissociation symptoms; and dependency are a problem, and because of its narcotic designation, clinical application presents difficulties.

Furthermore, it is known that within RS-ketamine, S-ketamine, which is the S-isomer, has an affinity toward NMDA receptors that is about four times higher than R-ketamine, which is the R-isomer (non-patent literature 11). Meanwhile, in animal testing, R-ketamine reportedly has fewer side effects (such as drug dependency and an action of inducing a mental illness) compared to S-ketamine, and an intensity of an action of inducing mental-illness symptoms by ketamines is correlated to a blocking intensity of NMDA receptors (non-patent literature 12 to 14).

Furthermore, both the analgesic action and the action of inducing mental-illness symptoms of RS-ketamine are generally understood to be mainly mediated by blocking NMDA receptors (non-patent literature 3); because the affinity of S-ketamine toward NMDA receptors is high, the actions of RS-ketamine are thought to be mainly caused by S-ketamine.

Although cognitive impairments are observed in patients of many mental illnesses, including neurodevelopmental disorders such as schizophrenia, autism spectrum disorder, learning disorders, and attention-deficit/hyperactivity disorder (non-patent literature 15), there are no drugs to prevent and treat these cognitive impairments.

### [Prior-Art Literature]

### [Non-Patent Literature]

[Non-Patent Literature 1] ElvevB. et al. Cognitive impairment in schizophrenia is the core of the disorder. Crit. Rev. Neurobiol. 2000;14 (1):1-21.
[Non-Patent Literature 2] Barch DM. et al. Cognition in schizophrenia: core psychological and neural mechanisms. Trend Cog. Sci. 2012 Jan;16(1):27-34.
[Non-Patent Literature 3] Javitt DC. et al. Recent advances in the phencyclidine model of schizophrenia. Am. J. Psychiatry 1991 Oct;148(10):1301-8.
[Non-Patent Literature 4] Domino EF. Et al. Phencyclidine/schizophrenia: one view toward the past, the other to the future. Schizophr. Bull. 2012 Sep; 38(5):914-919.
[Non-Patent Literature 5] Sams-Dodd F. Phencyclidine-induced stereotyped behavior and social isolation in rats: a possible animal model of schizophrenia. Behav. Pharmacol. 1996;7(1):3-23.
[Non-Patent Literature 6] Hashimoto K. et al. Phencyclidine-induced cognitive deficits in mice are improved by subsequent subchronic administration of clozapine, but not haloperidol. Eur. J. Pharmacol. 2005 Sep;519(1-2):114-117.
[Non-Patent Literature 7] Hashimoto K. et al. Phencyclidine-induced cognitive deficits in mice are improved by subsequent subchronic administration of fluvoxamine: role of sigma-1 receptors. Neuropsychopharmacology 2007 Mar; 32(3):514-521.
[Non-Patent Literature 8] Hashimoto K. et al. Phencyclidine-induced cognitive deficits in mice are improved by subsequent subchronic administration of the novel selective alpha-7 nicotinic receptor agonist SSR180711. Biol. Psychiatry 2008 Jan; 63(1):92-97.
[Non-Patent Literature 9] Abi-Saab WM. et al. The NMDA antagonist model for schizophrenia: promise and pitfalls. Pharmacopsychiatry 1998 Jul; 31(Suppl 2):104-109.
[Non-Patent Literature 10] Frohlich J. et al. Reviewing the ketamine model for schizophrenia. J. Psychopharmacol. 2014 Apr; 28(4):287-302.
[Non-Patent Literature 11] Ebert B. et al. Norketamine, the main metabolite of ketamine, is an non-competitive NMDA receptor antagonist in the rat cortex and spinal cord. Eur. J. Pharmacol. 1997 Aug; 333(1):99-104.
[Non-Patent Literature 12] Yang C. et al. HYPERLINK "https://www.ncbi.nlm.nih.gov/pubmed/26327690" R-ketamine: a rapid-onset and sustained antidepressant without psychotomimetic side effects. Transl. Psychiatry 2015 Sep 1; 5:e632.
[Non-Patent Literature 13] Yang C. et al. Loss of parvalbumin-immunoreactivity in mouse brain regions after repeated intermittent administration of esketamine, but not R-ketamine. Psychiatry Res. 2016 May 30;239:281-3.
[Non-Patent Literature 14] Hashimoto K. et al. Reduction of dopamine D2/3 receptor binding in the striatum after a single administration of esketamine, but not R-ketamine: A PET study in conscious monkeys. Eur. Arch. Psychiatry Clin. Neurosci. 2017 March;267(2):173-176.
[Non-Patent Literature 15] Millan MJ. et al. Cognitive dysfunction in psychiatric disorders: characteristics, causes and the quest for improved therapy. Nature Rev. Drug Discov. 2012 Feb;11(2):141-168.

### [Summary of Invention]

### [Problem to Be Solved by Invention]

A problem to be solved by the present invention is to provide a new compound having a preventive or therapeutic effect regarding a neurodevelopmental disorder.

### [Means for Solving Problem]

The present inventors undertook intensive study to solve the above problem and found a new compound having a preventive or therapeutic effect regarding a neurodevelopmental disorder, thereby completing the present invention.

That is, the present invention is as follows.
(1) A preventive or therapeutic agent for a neurodevelopmental disorder that includes the compound illustrated in formula (I) below, or a pharmaceutically acceptable salt thereof, as an active ingredient.
   Formula (I): 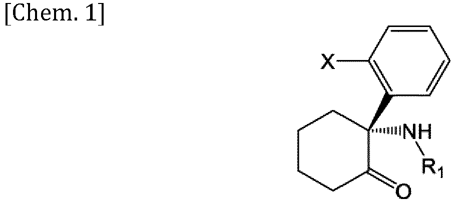 (In formula [I],
   X is H, F, Cl, Br, I, an alkyl, an alkenyl, or an aryl, and
   R1 is an alkyl, an alkenyl, or an aryl.)
(2) The preventive or therapeutic agent for a neurodevelopmental disorder of (1), wherein the preventive or therapeutic agent includes R-ketamine or a pharmaceutically acceptable salt thereof.
(3) The preventive or therapeutic agent for a neurodevelopmental disorder of (1) or (2), wherein the neurodevelopmental disorder is schizophrenia, autism spectrum disorder, attention-deficit/hyperactivity disorder, or a learning disorder-preferably schizophrenia or autism spectrum disorder and more preferably schizophrenia.
(4) The preventive agent or therapeutic agent for a neurodevelopmental disorder of any one among (1) to (3), wherein the neurodevelopmental disorder is a childhood neurodevelopmental disorder.
(5) The preventive or therapeutic agent for a neurodevelopmental disorder of any one among (1) to (3), wherein the neurodevelopmental disorder is a fetal or infantile neurodevelopmental disorder.
(6) The preventive agent or therapeutic agent for a neurodevelopmental disorder of any one among (1) to (5), wherein the neurodevelopmental disorder is related to inflammation of a mother during pregnancy.
(7) The preventive or therapeutic agent for a neurodevelopmental disorder of any one among (1) to (6), wherein the preventive or therapeutic agent is used to prevent or treat a cognitive impairment or a negative symptom-preferably used for a cognitive impairment or negative symptom based on a neurodevelopmental disorder and more preferably used for a cognitive impairment or negative symptom based on schizophrenia.
(8) The preventive or therapeutic agent for a neurodevelopmental disorder of (7), wherein the cognitive impairment includes impaired attention, reduced linguistic fluidity, reduced learning and retention of linguistic information, reduced processing speed, reduced declarative memory, impaired working memory, a reduced executive function, or a combination thereof.
(9) The preventive or therapeutic drug for a neurodevelopmental disorder of (7), wherein the negative symptom includes social withdrawal, lack of social interest, anhedonia, blunted affect, indifference or inattention toward social or cognitive input, or a combination thereof.
(10) The preventive or therapeutic agent for a neurodevelopmental disorder of any one among (1) to (9), wherein the preventive or therapeutic agent substantially does not include the compound illustrated in formula (II) below or a pharmaceutically acceptable salt thereof.
   Formula (II): 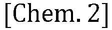 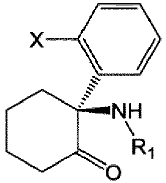 (In formula [II],
   definitions of X and R1 are similar to the definitions in formula [I].)
(11) The preventive or therapeutic agent for a neurodevelopmental disorder of any one among (1) to (10), wherein the preventive or therapeutic agent substantially does not include S-ketamine or a pharmaceutically acceptable salt thereof.
(12) The preventive or therapeutic agent for a neurodevelopmental disorder of (11), wherein an amount of S-ketamine or a pharmaceutically acceptable salt thereof is no greater than 2% by weight or no greater than 0.15% by weight.
(13) The preventive or therapeutic agent for a neurodevelopmental disorder of (11) or (12), wherein an amount of S-ketamine or a pharmaceutically acceptable salt thereof is an amount of S-ketamine or a pharmaceutically acceptable salt thereof whereat no side effect of S-ketamine or a pharmaceutically acceptable salt thereof is exhibited.
(14) The preventive or therapeutic agent for a neurodevelopmental disorder of (13), wherein the side effect is a psychotogenetic action.
(15) A preventive or therapeutic agent for a neurodevelopmental disorder made of the compound illustrated in formula (I) above, or a pharmaceutically acceptable salt thereof, as an active ingredient.
(16) A preventive or therapeutic agent for a neurodevelopmental disorder made of R-ketamine or a pharmaceutically acceptable salt thereof.
(17) A pharmaceutical composition, including: the preventive or therapeutic agent for a neurodevelopmental disorder of any one among (1) to (16).
(18) The pharmaceutical composition of (17), wherein the pharmaceutical composition is suited for intravenous, intramuscular, subcutaneous, nasal, oral, sublingual, rectal, or percutaneous administration.
(19) The pharmaceutical composition of (17) or (18), wherein the pharmaceutical composition is in the form of a liquid, a solution, a suspension, a powder, a tablet, a coated tablet, a capsule, a troche, a cream, a suppository, a gel, a patch, a liniment, or an aerosol.
(20) The pharmaceutical composition of any one among (17) to (19), wherein the pharmaceutical composition includes an amount of R-ketamine that is effective in treating a neurodevelopmental disorder.
(21) A preventive or therapeutic agent for a cognitive impairment in a patient that includes the compound illustrated in formula (I) below or a pharmaceutically acceptable salt thereof.
   Formula (I): 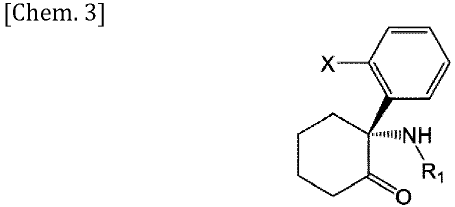 (In formula [I],
   X is H, F, Cl, Br, I, an alkyl, an alkenyl, or an aryl, and
   R1 is an alkyl, an alkenyl, or an aryl.)
(22) The preventive or therapeutic agent for a cognitive impairment of (21), wherein the preventive or therapeutic agent includes R-ketamine or a pharmaceutically acceptable salt thereof.
(23) The preventive or therapeutic agent for a cognitive impairment of (21) or (22), wherein the preventive or therapeutic agent substantially does not include the compound illustrated in formula (II) below or a pharmaceutically acceptable salt thereof. Formula (II): 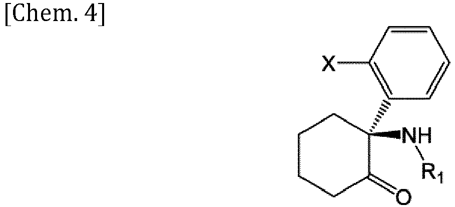 (In formula [II], definitions of X and R1 are similar to the definitions in formula [I].)
(24) The preventive or therapeutic agent for a cognitive impairment of any one among (21) to (23), wherein the preventive or therapeutic agent substantially does not include S-ketamine or a pharmaceutically acceptable salt thereof.
(25) The preventive or therapeutic agent for a cognitive impairment of (24), wherein an amount of S-ketamine or a pharmaceutically acceptable salt thereof is no greater than 2% by weight or no greater than 0.15% by weight.
(26) The preventive or therapeutic agent for a cognitive impairment of (24) or (25), wherein an amount of S-ketamine or a pharmaceutically acceptable salt thereof is an amount of S-ketamine or a pharmaceutically acceptable salt thereof whereat no side effect of S-ketamine or a pharmaceutically acceptable salt thereof is exhibited.
(27) The preventive or therapeutic agent for a cognitive impairment of (26), wherein the side effect is a psychotogenetic action.
(28) The preventive or therapeutic agent for a cognitive impairment of any one among (21) to (27), wherein the cognitive impairment stems from a neurodevelopmental disorder.
(29) The preventive agent or therapeutic agent for a cognitive impairment of (28), wherein the neurodevelopmental disorder is a childhood neurodevelopmental disorder.
(30) The preventive or therapeutic agent for a cognitive impairment of (28), wherein the neurodevelopmental disorder is a fetal or infantile neurodevelopmental disorder.
(31) The preventive or therapeutic agent for a cognitive impairment of any one among (28) to (30), wherein the neurodevelopmental disorder is schizophrenia, autism spectrum disorder, a learning disorder, or attention-deficit/hyperactivity disorder.
(32) The preventive agent or therapeutic agent for a cognitive impairment of any one among (28) to (30), wherein the neurodevelopmental disorder is related to inflammation of a mother during pregnancy.
(33) The preventive or treatment agent for a cognitive impairment of any one among (21) to (32), wherein the cognitive impairment includes impaired attention, reduced linguistic fluidity, reduced learning and retention of linguistic information, reduced processing speed, reduced declarative memory, impaired working memory, a reduced executive function, or a combination thereof.
(34) A pharmaceutical composition, including: the preventive or therapeutic agent for a cognitive impairment of any one among (21) to (33); and a pharmaceutically acceptable carrier, diluent, or excipient.
(35) The pharmaceutical composition of (34), wherein the pharmaceutical composition is suited for intravenous, intramuscular, subcutaneous, nasal, oral, sublingual, rectal, or percutaneous administration.
(36) The pharmaceutical composition of (34) or (35), wherein the pharmaceutical composition is in the form of a liquid, a solution, a suspension, a powder, a tablet, a coated tablet, a capsule, a troche, a cream, a suppository, a gel, a patch, a liniment, or an aerosol.
(37) The pharmaceutical composition of any one among (34) to (36), wherein the pharmaceutical composition includes an amount of R-ketamine that is effective in treating a cognitive impairment.
(38) A preventive or therapeutic pharmaceutical composition for a neurodevelopmental disorder that contains the compound illustrated in formula (I) above, or a pharmaceutically acceptable salt thereof, at an amount that is effective in mitigating a symptom of a neurodevelopmental disorder and substantially does not include the compound illustrated in formula (II) above or a pharmaceutically acceptable salt thereof.
(39) A preventive or therapeutic pharmaceutical composition for a neurodevelopmental disorder that contains R-ketamine, or a pharmaceutically acceptable salt thereof, at an amount that is effective in mitigating a symptom of a neurodevelopmental disorder and substantially does not include S-ketamine or a pharmaceutically acceptable salt thereof.
(40) The compound illustrated in formula (I) above, or a pharmaceutically acceptable salt thereof, wherein the compound or a pharmaceutically acceptable salt thereof is for preventing or treating a neurodevelopmental disorder.
(42) R-ketamine, or a pharmaceutically acceptable salt thereof, wherein R-ketamine or a pharmaceutically acceptable salt thereof is for preventing or treating a neurodevelopmental disorder.
(43) A method of preventing or treating a neurodevelopmental disorder, including the step of: administering the compound illustrated in formula (I) above or a pharmaceutically acceptable salt thereof to a patient needing such.
(44) A method of preventing or treating a neurodevelopmental disorder, including the step of: administering R-ketamine or a pharmaceutically acceptable salt thereof to a patient needing such.
(45) In (38) to (44), "neurodevelopmental disorder" may be replaced with "positive symptom, negative symptom, and/or cognitive impairment of neurodevelopmental disorder." The neurodevelopmental disorder may be schizophrenia, autism spectrum disorder, attention-deficit/hyperactivity disorder, or a learning disorder. Moreover, the neurodevelopmental disorder may be a cognitive impairment due to a neurodevelopmental disorder.

### [Effects of Invention]

The compound illustrated in formula (I), a representative thereof being R-ketamine, or a pharmaceutically acceptable salt thereof is effective in preventing or treating a neurodevelopmental disorder.

### [Brief Description of Drawings]

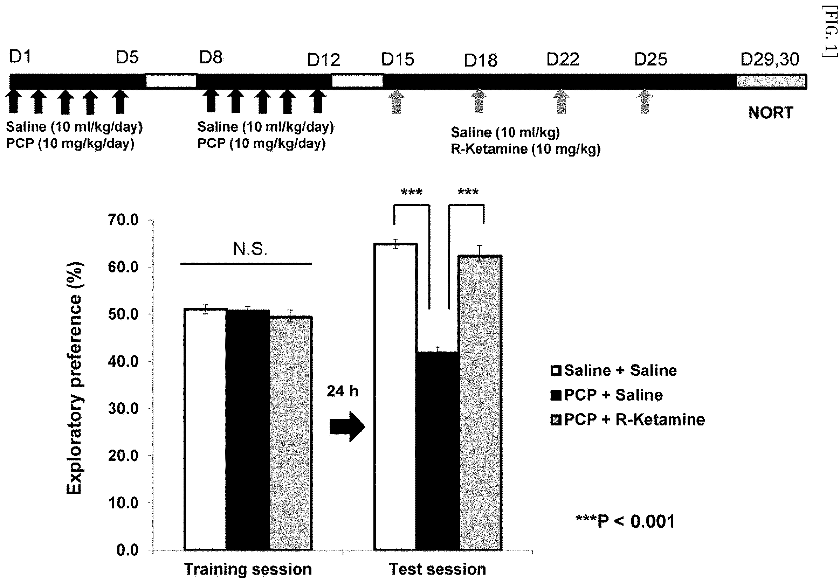

[FIG. 1] A diagram for describing a testing plan whereby an effect of R-ketamine on model animals of schizophrenia are examined. Illustrated are results of a novel object recognition test for a group administered with physiological saline + physiological saline, a group administered with PCP + physiological saline, and a group administered with PCP + R-ketamine.

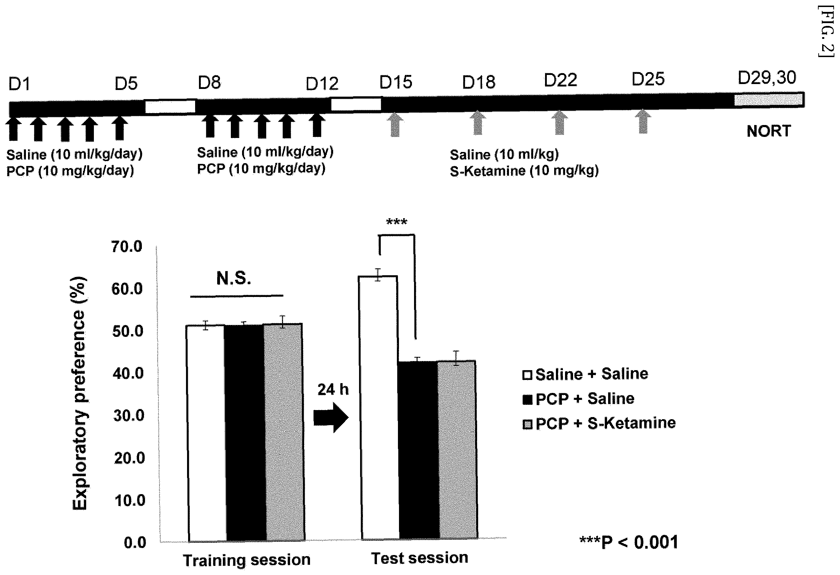

[FIG. 2] A diagram for describing a testing plan whereby an effect of S-ketamine on model animals of schizophrenia are examined. Illustrated are results of a novel object recognition test for a group administered with physiological saline + physiological saline, a group administered with PCP + physiological saline, and a group administered with PCP + S-ketamine.

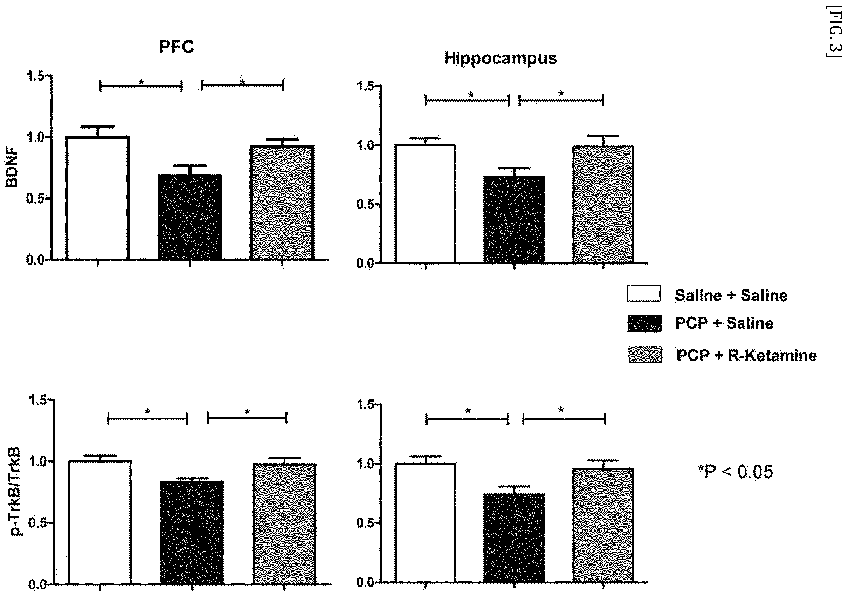

[FIG. 3] Illustrated are quantitative results of brain-derived neurotrophic factor (BDNF: brain-derived neurotrophic factor); TrkB, which is a BDNF receptor; and phosphorylated TrkB in extracted frontal cortices and hippocampi.

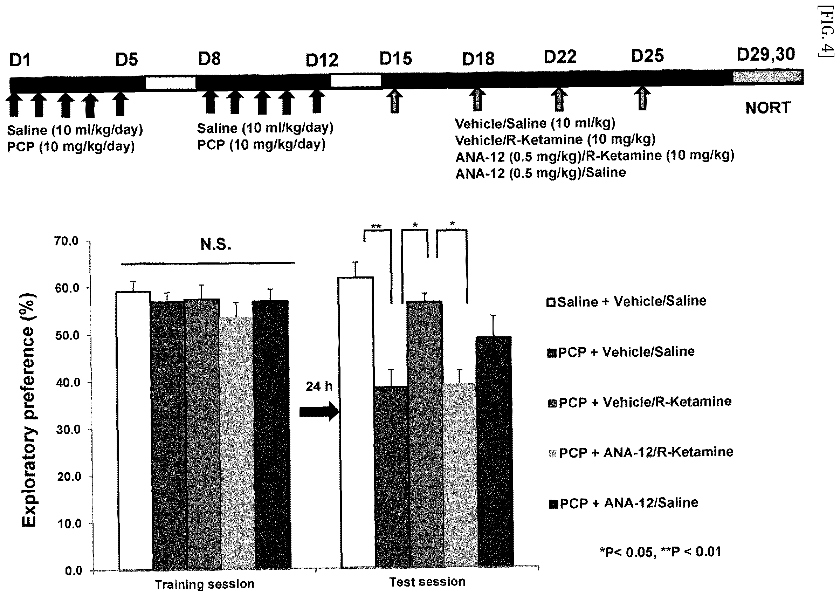

[FIG. 4] A diagram for describing a testing plan whereby an effect of R-ketamine on model animals of schizophrenia are examined. Illustrated are results of a novel object recognition test for a group administered with physiological saline + a solvent / physiological saline, a group administered with PCP + the solvent / physiological saline, a group administered with PCP + the solvent / R-ketamine, a group administered with PCP + ANA-12/R-ketamine, and a group administered with PCP + ANA-12 / physiological saline.

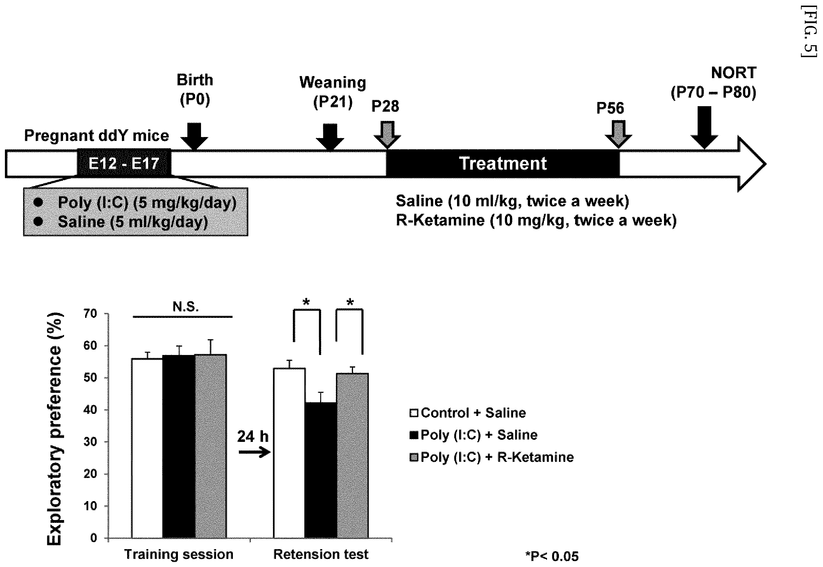

[FIG. 5] A diagram for describing a testing plan whereby an effect of R-ketamine on model animals of a neurodevelopmental disorder are examined. Illustrated are results of a novel object recognition test for a group administered with a control + physiological saline, a group administered with Poly (I:C) + physiological saline, and a group administered with Poly (I:C) + R-ketamine.

### [Embodiments of Invention]

A preventive or therapeutic agent for a neurodevelopmental disorder of the present invention includes the compound illustrated in formula (I) below, or a pharmaceutically acceptable salt thereof, as an active ingredient (hereinbelow, "compound illustrated in formula (I)" may simply be referred to as "compound I").

### Formula (I):

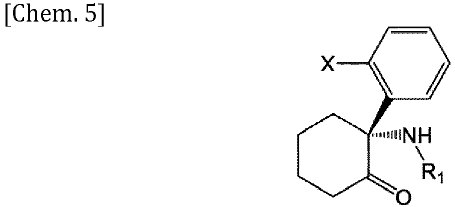

(In formula [I],
X is H, F, Cl, Br, I, an alkyl, an alkenyl, or an aryl, and
R1 is an alkyl, an alkenyl, or an aryl.)

X is H, F, Cl, BR, I, an alkyl, an alkenyl, or an aryl-preferably F or Cl and more preferably Cl.

R1 is an alkyl, an alkenyl, or an aryl.

In the present invention, a carbon number of an alkyl and an alkenyl is preferably 1 to 10, more preferably 1 to 6, and even more preferably 1 to 5.

When X and R1 are an alkyl, while not limited in particular, for example, methyl, ethyl, propyl, isopropyl, cyclopropyl, butyl, isobutyl, t-butyl, cyclobutyl, cyclopropylmethyl, pentyl, and cyclopentyl can be mentioned; the alkyl is preferably a linear alkyl, more preferably a linear alkyl of a carbon number of 1 to 5, even more preferably methyl or ethyl, and even still more preferably methyl.

When X and R1 are an alkenyl, this may be an alkenyl that is a group having one double bond or no fewer than two double bonds with a group listed as an alkyl; while not limited in particular, for example, vinyl and allyl can be mentioned.

When X and R1 are an aryl, while not limited in particular, for example, phenyl can be mentioned.

In formula (I), a compound wherein X is Cl and R1 is methyl is R-ketamine.

The preventive or therapeutic agent for a neurodevelopmental disorder in the present invention preferably includes R-ketamine, or a pharmaceutically acceptable salt thereof, as an active ingredient.

The present invention relates to a preventive or therapeutic drug for a neurodevelopmental disorder made of compound I or a pharmaceutically acceptable salt thereof. Moreover, the present invention relates to a preventive or therapeutic pharmaceutical composition for a neurodevelopmental disorder that contains compound I, or a pharmaceutically acceptable salt thereof, at an amount that is effective in mitigating a symptom of a neurodevelopmental disorder and substantially does not include the compound illustrated in formula (II) or a pharmaceutically acceptable salt thereof (hereinbelow, "compound illustrated in formula (II)" may simply be referred to as "compound II").

### Formula (II):

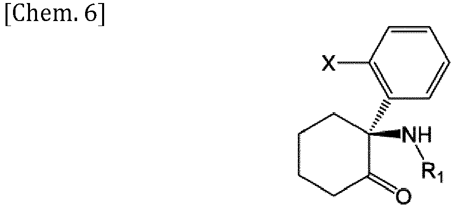

(In formula [II],
definitions of X and R1 are similar to the definitions in formula [I].)

In the present invention, the phrase "as an active ingredient" signifies inclusion as a principal active ingredient; as long as compound I or a pharmaceutically acceptable salt thereof is included as a medicinal ingredient, a content thereof is not limited in particular.

When compound I or a pharmaceutically acceptable salt thereof is included as an active ingredient, preferably, compound II or a pharmaceutically acceptable salt thereof is substantially not included.

In the present invention, the phrase "substantially does not include the compound illustrated in formula (II) or a pharmaceutically acceptable salt thereof" signifies that compound II or a pharmaceutically acceptable salt thereof is not included at all, that compound II or a pharmaceutically acceptable salt thereof may be included at an amount whereat an effect or side effect thereof does not arise, or that this may be included as an impurity to an extent of being unavoidably mixed in during manufacturing.

Furthermore, the phrase "substantially does not include the compound illustrated in formula (II) or a pharmaceutically acceptable salt thereof" may signify that the "compound illustrated in formula (II) or a pharmaceutically acceptable salt thereof" may be included as an impurity, according to the "Guidelines Relating to API Impurities in Pharmaceutical Products Containing Novel Active Ingredients," with regard to the compound illustrated in formula (I), or a pharmaceutically acceptable salt thereof, which is the API when a pharmaceutical product is provided; compound II may be included at no greater than 0.15% in the API.

In the present invention, "compound illustrated in formula (I)" in "compound illustrated in formula (I) or a pharmaceutically acceptable salt thereof" is preferably R-ketamine, and "compound illustrated in formula (II)" in "compound illustrated in formula (II) or a pharmaceutically acceptable salt thereof" is preferably S-ketamine.

Note that RS-ketamine is a racemate that is an equal mixture of R-ketamine and S-ketamine.

Preferably, the preventive or therapeutic agent for a neurodevelopmental disorder of the present invention substantially does not include the compound illustrated in formula (II) or a pharmaceutically acceptable salt thereof, but in this situation, an amount of the compound illustrated in formula (II) or a pharmaceutically acceptable salt thereof, or an amount of S-ketamine or a pharmaceutically acceptable salt thereof, is preferably no greater than 2% by weight and preferably no greater than 0.15% by weight. The amount of the compound illustrated in formula (II) or a pharmaceutically acceptable salt thereof, or the amount of S-ketamine or a pharmaceutically acceptable salt thereof, may be 0% by weight, no less than 0% by weight, or an amount in excess of 0% by weight. The amount of the compound illustrated in formula (II) or a pharmaceutically acceptable salt thereof, or the amount of S-ketamine or a pharmaceutically acceptable salt thereof, may be a content in a total amount of the preventive or therapeutic agent for a neurodevelopmental disorder, and "% by weight" may be replaced with "% by mass."

Furthermore, preferably, the preventive or therapeutic agent for a neurodevelopmental disorder of the present invention substantially does not include the compound illustrated in formula (II) or a pharmaceutically acceptable salt thereof, but in this situation, the amount of the compound illustrated in formula (II) or a pharmaceutically acceptable salt thereof, or the amount of S-ketamine or a pharmaceutically acceptable salt thereof, may be an amount of the compound illustrated in formula (II) or a pharmaceutically acceptable salt thereof whereat a side effect of the compound illustrated in formula (II) or a pharmaceutically acceptable salt thereof is not exhibited, or an amount of S-ketamine or a pharmaceutically acceptable salt thereof whereat a side effect of S-ketamine or a pharmaceutically acceptable salt thereof is not exhibited.

"Side effect" as referred to here may be a mental-illness inducing action, drug dependency, or the like or a psychotogenetic action.

In the present invention, the term "neurodevelopmental disorder" is a group of diseases arising due to a flaw in a developmental period (such as the fetal period, infancy, childhood, or puberty) of the nervous system.

As the neurodevelopmental disorder, while not limited in particular, for example, schizophrenia, autism spectrum disorder, attention-deficit/hyperactivity disorder, and a learning disorder can be mentioned.

The neurodevelopmental disorder is preferably schizophrenia.

Here, as one cause of the neurodevelopmental disorder, as a result of epidemiological investigation, involvement of maternal inflammation can be mentioned. Therefore, the neurodevelopmental disorder may be a neurodevelopmental disorder related to inflammation of a mother during pregnancy.

In schizophrenia, which is one neurodevelopmental disorder, symptoms that are positive symptoms such as hallucination and delusion; negative symptoms such as blunted or flat affect, diminished volition, reduced thinking, and social withdrawal; and cognitive impairments such as reduced attention, reduced working memory, and a reduced executive function are confirmed.

The preventive or therapeutic agent for a neurodevelopmental disorder in the present invention can be used to prevent or treat a positive symptom, a negative symptom, and/or a cognitive impairment of a neurodevelopmental disorder.

The negative symptom may be, in addition to the above, for example, social withdrawal, lack of social interest, anhedonia, blunted affect, indifference or inattention toward social or cognitive input, or a combination thereof.

Furthermore, the cognitive impairment may be, in addition to the above, for example, impaired attention, reduced linguistic fluidity, reduced learning and retention of linguistic information, reduced processing speed, reduced declarative memory, impaired working memory, a reduced executive function, or a combination thereof.

Among these, the preventive or therapeutic agent for a neurodevelopmental disorder in the present invention may be used to prevent or treat a cognitive impairment or negative symptom above and is preferably used to prevent or treat a cognitive impairment or negative symptom based on schizophrenia.

In the present invention, compound I or a pharmaceutically acceptable salt thereof has a neurotrophic-factor action. As such, it may be used to prevent a neurodevelopmental disorder by being administered before various symptoms of the neurodevelopmental disorder appear, and it may be used to treat a cognitive impairment or a negative symptom of a neurodevelopmental disorder by an action of mitigating nerve-cell loss involved in the neurodevelopmental disorder.

Furthermore, from many epidemiological studies, it is known that children born under maternal immune activation (maternal inflammation) have a high risk of developing a neurodevelopmental disorder. According to pharmaceutical test results using mouse models using maternal immune activation, R-ketamine can serve as a preventive or therapeutic agent for a neurodevelopmental disorder.

The preventive or therapeutic agent for a neurodevelopmental disorder of the present invention is not limited in particular but may be a neurodevelopmental disorder of a developmental period (such as the fetal period, infancy, childhood, or puberty) of the nervous system-in particular, of the fetal period, infancy, or childhood (fetal, infantile, or childhood neurodevelopmental disorder).

Additionally, improving a cognitive impairment that is a core symptom of the neurodevelopmental disorder-preferably, schizophrenia-can also lead to improving a positive symptom of the neurodevelopmental disorder-preferably, schizophrenia.

By using the preventive or therapeutic agent for a neurodevelopmental disorder in the present invention, a neurodevelopmental disorder is prevented from appearing, and a therapeutic effect such as a mitigated or improved symptom is exhibited in a patient with a neurodevelopmental disorder.

Neurodevelopmental disorders are symptoms that are present over a long period (in year units). As such, by starting treatment early, progression of the symptoms can also be prevented, and it is anticipated that such will enable social rehabilitation for a patient.

In the present invention, the compound illustrated in formula (I) or a pharmaceutically acceptable salt thereof is used to prevent or treat a neurodevelopmental disorder. As such, administration over a long period is planned.

Therefore, using a compound illustrated in formula (I) that is an R-body, or a pharmaceutically acceptable salt thereof, enables administration over a long period without a side effect being exhibited. This enables the compound illustrated in formula (I) or a pharmaceutically acceptable salt thereof to be used as a preventive or therapeutic agent for a neurodevelopmental disorder.

The preventive agent for a neurodevelopmental disorder in the present invention may be an agent that prevents the appearance of a neurodevelopmental disorder or an agent that prevents the progression of a symptom. Moreover, the therapeutic agent for a neurodevelopmental disorder in the present invention has therapeutic effects such as preventing the progression of a symptom and mitigating or improving a symptom.

The compound illustrated in formula (I), which is an active ingredient in the present invention, can be used in both the form of a free base and the form of a pharmaceutically acceptable salt thereof.

The following description is given using R-ketamine as an example. Matters described concerning R-ketamine can also be applied to the compound illustrated in formula (I). In this situation, locations written as "S-ketamine" can be replaced with "compound illustrated in formula (11)."

R-ketamine is a free base and has the chemical structure illustrated in formula (III) below.

### Formula (III):

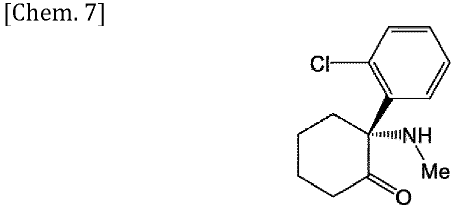

As a pharmaceutically acceptable salt of R-ketamine, an addition salt of a pharmaceutically acceptable acid is preferable; the pharmaceutically acceptable acid is not limited in particular but is preferably hydrochloric acid.

R-ketamine hydrochloride has the chemical structure illustrated in formula (IV) below.

### Formula (IV):

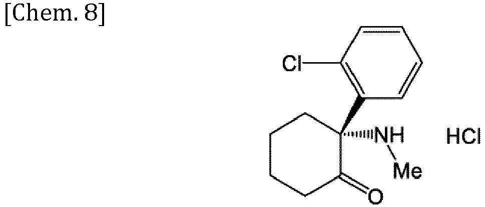

R-ketamine, or a pharmaceutically acceptable salt thereof, used in the present invention may be in the form of a hydrate or in the form of a solvate.

Furthermore, R-ketamine or a pharmaceutically acceptable salt thereof may be isotope-labeled.

An isotope is not limited in particular, but, for example, 13C and 2H(D), which are stable isotopes, can be mentioned.

By performing isotope labeling, the in vivo kinetics of R-ketamine, or a pharmaceutically acceptable salt thereof, before isotope labeling can be changed. For example, the metabolism of R-ketamine hydrochloride labeled with heavy hydrogen, which has the chemical structure illustrated in formula (V) below (in formula [V], D represents a heavy hydrogen atom), is thought to become slower, and it is anticipated that a duration will be lengthened.

### Formula (V):

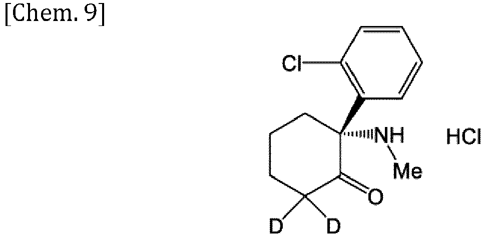

The preventive or therapeutic agent, drug, and pharmaceutical composition for a neurodevelopmental disorder in the present invention can be administered orally or non-orally.

Oral administration can use a known dosage form for administration such as a tablet (including a coated tablet); a capsule; a coated tablet; a troche; or a liquid agent such as a liquid, a solution, or a suspension. Oral administration may be sublingual administration. Moreover, as non-oral administration, intravenous, intramuscular, or subcutaneous administration by injection; transmucosal administration via the nasal cavity, the oral cavity, or the like using a powder, drops, a spray, an aerosol, or the like; rectal administration using a cream, a suppository, or the like; percutaneous administration using a patch, a liniment, a gel, or the like; and the like can be mentioned.

The route of administration is preferably percutaneous administration; oral administration; nasal administration; or intravenous administration, subcutaneous administration, or intramuscular administration by injection.

In addition to R-ketamine or a pharmaceutically acceptable salt thereof, the pharmaceutical composition in the present invention may include another medicinal ingredient with a neuroprotective effect-other than S-ketamine or a pharmaceutically acceptable salt thereof-or another medicinal ingredient that can be used to treat a neurodevelopmental disorder.

Furthermore, in addition to these medicinal ingredients, the pharmaceutical composition may include, as appropriate and according to the dosage form and the like, a pharmaceutically acceptable additive that is well-known by a person skilled in the art.

The pharmaceutically acceptable additive is not limited in particular, but, for example, an antioxidant, a stabilizer, a preservative, a flavoring agent, a colorant, a solvent, a solubilizer, a surfactant, an emulsifier, a defoamer, a viscosity modifier, a gelling agent, an absorption promoter, a dispersant, a carrier, a diluent, an excipient, and a pH adjuster can be mentioned.

When preparing the preventive or therapeutic agent, drug, and pharmaceutical composition for a neurodevelopmental disorder in the present invention as a preparation for injection, a preparation form of a solution or a suspension is preferable. When preparing this as a preparation for transmucosal administration via the nasal cavity, the oral cavity, or the like, a preparation form of a powder, drops, or an aerosol is preferable. When preparing this as a preparation for rectal administration, a preparation form of a semisolid preparation such as a cream or a suppository is preferable.

Any of the preparations administered orally or non-orally can be prepared by any method known to a person skilled in pharmaceutical art, such as a method described in Remington's Pharmaceutical Sciences (Mack Publishing Co., Easton, PA, 1970).

As a carrier, the preparation for injection may use, for example, a plasma-derived protein such as albumin, an amino acid such as glycine, or a sugar such as mannitol. Moreover, a buffer, a solubilizing agent, an isotonic agent, or the like may be used. Moreover, in use as a water-soluble preparation or a freeze-dried preparation, to prevent clumping, a surfactant such as Tween (registered trademark) 80 or Tween (registered trademark) 20 may be used.

As a carrier, a preparation for non-oral administration other than a preparation for injection may use, for example, distilled water or physiological saline, a polyalkylene glycol such as polyethylene glycol, an oil of vegetable origin, or hydrogenated naphthalene. For example, a preparation for rectal administration such as a suppository may use, for example, polyalkylene glycol, Vaseline, or cacao fat as an excipient. A preparation for vaginal administration may use an absorption promoter such as a bile salt, an ethylenediamine salt, or a citric-acid salt as a carrier. A preparation for inhalation may be a solid, and as an excipient, for example, lactose may be used. Drops for nasal administration may be an aqueous or oil solution.

A precise dosage and dosage regimen of the preventive or therapeutic agent, drug, and pharmaceutical composition for a neurodevelopmental disorder in the present invention is adjusted depending on the required amount, the treatment method, the disease, the extent of necessity, and the like of each individual treatment target.

Specifically, the dosage can be determined according to age, bodyweight, overall health, sex, meals, administration time, administration method, excretion rate, drug combination, patient symptoms, and the like and may also be determined in consideration of other factors.

When administering the preventive or therapeutic agent, drug, and pharmaceutical composition for a neurodevelopmental disorder in the present invention to a patient having a cognitive impairment or a negative symptom of a neurodevelopmental disorder, preferably, R-ketamine or a pharmaceutically acceptable salt thereof is included at an amount that, preferably, is effective in mitigating the cognitive impairment or the negative symptom of the neurodevelopmental disorder.

R-ketamine or a pharmaceutically acceptable salt thereof has few of the side effects recognized for S-ketamine and RS-ketamine and can therefore be safely used. A dosage for one day varies according to the state and bodyweight of the patient, the type of compound, the route of administration, and the like but is normally about 0.01 to 1,000 mg/person/day and preferably 0.1 to 500 mg/person/day in non-oral administration and normally about 0.01 to 500 mg/person/day and preferably 0.1 to 100 mg/person/day in oral administration.

The present invention may be a method of preventing or treating a neurodevelopmental disorder, including the step of administering compound I or a pharmaceutically acceptable salt thereof to a patient needing such.

The patient needing such is not limited in particular and is a mammal-preferably a human.

Compound 1 or a pharmaceutically acceptable salt thereof is preferably administered at a therapeutically effective amount.

### [Examples]

The present invention is described more specifically below by examples, but the present invention is not limited to the following examples. Moreover, various modifications are possible within a scope that does not depart from the technical idea of the present invention.

All testing was conducted with consent from the Chiba University Animal Testing Committee.

Using animal models of schizophrenia by PCP administration, which has hitherto been used in many research groups, therapeutic effects of R-ketamine and S-ketamine on a cognitive impairment of the model animals were examined.

R-ketamine hydrochloride and S-ketamine hydrochloride were prepared from RS-ketamine (Ketalar [registered trademark], ketamine hydrochloride, Daiichi Sankyo Co. Ltd., Tokyo, Japan) using D-tartaric acid or L-tartaric acid by the method described in US 6040479 A. A purity of each isomer was confirmed by high-performance liquid chromatography (CHIRALPAK [registered trademark] IA, column size: 250 × 4.6 mm, mobile phase; n-hexane/dichloromethane/diethylamine (75/25/0.1), holding time of S-ketamine = 6.99 minutes, holding time of R-ketamine = 10.56 minutes, Daicel Corp., Tokyo, Japan).

The creation of model animals of a cognitive impairment of schizophrenia and drug administration were specifically carried out as described below (FIG. 1). For the mice, male ICR mice (8 weeks old, Japan SLC Inc., Hamamatsu, Japan) were purchased. The mice were allowed to freely ingest water and feed. On the first day of testing, physiological saline (10 mL / kg bodyweight) or PCP (10 mg / kg bodyweight) was subcutaneously administered once a day for ten days (from the first day of testing to the fifth day of testing and from the eighth day of testing to the twelfth day of testing: no administration on the sixth and seventh days of testing). On the fifteenth day of testing, the eighteenth day of testing, the twenty-second day of testing, and the twenty-fifth day of testing, R-ketamine (10 mg / kg bodyweight) or a medium (10 mL / kg of physiological saline) was intraperitoneally administered. This resulted in (A) a group administered with physiological saline + physiological saline, (B) a group administered with PCP + physiological saline, and (C) a group administered with PCP + R-ketamine.

The novel object recognition test (NORT: novel object recognition test) is a system used to evaluate cognitive functions. The NORT was carried out according to a previous report (Hashimoto et al. Eur.J. Pharmacol. 2005; 519 (1-2): 114-117). From the twenty-sixth day to the twenty-eighth day of testing, acclimation to a NORT apparatus (50.8 × 50.8 × 25.4 cm) was performed for 10 minutes per day. On the twenty-ninth day of testing, two objects of different shapes and colors were placed 35.5 cm apart in the NORT apparatus, and an amount of time to find each object was measured. A training session (10 minutes) was carried out, and on the following day, the thirtieth day of testing, a test session (5 minutes) was carried out (FIG. 1). In the test session on the following day, one object was replaced with another, new object (novel object). An amount of time to find the two objects was measured, and in the test session on the second day, a ratio of an amount of time to find the novel object relative to the amount of time to find the two objects was calculated.

On the day following the end of behavior testing, a brain region (frontal cortex, hippocampus) of the mice was extracted, and the brain tissue was stored in a freezer. Brain-derived neurotrophic factor (BDNF: brain-derived neurotrophic factor); TrkB, which is a BDNF receptor; and phosphorylated TrkB in the brain tissue were quantified by western blotting (FIG. 3).

The effect of S-ketamine was examined according to the method above. On the first day of testing, physiological saline (10 mL / kg bodyweight) or PCP (10 mg / kg bodyweight) was subcutaneously administered once a day for ten days (from the first day of testing to the fifth day of testing and from the eighth day of testing to the twelfth day of testing: no administration on the sixth and seventh days of testing). On the fifteenth day of testing, the eighteenth day of testing, the twenty-second day of testing, and the twenty-fifth day of testing, S-ketamine (10 mg / kg bodyweight) or a medium (10 mL / kg of physiological saline) was intraperitoneally administered. This resulted in (A) a group administered with physiological saline + physiological saline, (B) a group administered with PCP + physiological saline, and (C) a group administered with PCP + S-ketamine. The NORT was carried out according to the method above.

According to the method above, the role of TrkB receptors on the effect of R-ketamine was examined. On the first day of testing, physiological saline (10 mL / kg bodyweight) or PCP (10 mg / kg bodyweight) was subcutaneously administered once a day for ten days (from the first day of testing to the fifth day of testing and from the eighth day of testing to the twelfth day of testing: no administration on the sixth and seventh days of testing). On the fifteenth day of testing, the eighteenth day of testing, the twenty-second day of testing, and the twenty-fifth day of testing, a solvent (10 mL / kg bodyweight of PBS including 17% DMSO) / physiological saline (10 mL / kg bodyweight), the solvent (10 mL / kg bodyweight) / R-ketamine (10 mg / kg bodyweight), ANA-12 (0.5 mg / kg bodyweight) / R-ketamine (10 mg / kg bodyweight), or ANA-12 (0.5 mg / kg bodyweight) / physiological saline (10 mL / kg bodyweight) was intraperitoneally administered. This resulted in (A) a group administered with physiological saline + the solvent / physiological saline, (B) a group administered with PCP + the solvent / physiological saline, (C) a group administered with PCP + the solvent / R-ketamine, (D) a group administered with PCP + ANA-12/R-ketamine, and (C) a group administered with PCP + ANA-12 / physiological saline. The solvent or ANA-12 is intraperitoneally administered 30 minutes before administering R-ketamine or physiological saline. The NORT was carried out according to the method above.

Statistical analysis was carried out by performing a one-way analysis of variance (one-way ANOVA) and subsequently performing Fisher's LSD (Fisher's LSD). Data in FIG. 1 are represented as average ± standard error (n = six to eight mice / group). Data in FIG. 2 are represented as average ± standard error (n = seven to nine mice / group). Data in FIG. 3 are represented as average ± standard error (n = ten to twelve mice / group). Data in FIG. 4 are represented as average ± standard error (n = six to nine mice / group). "*** p < 0.001" indicates the significant difference compared to the mouse group treated with PCP + physiological saline.

A significant reduction in cognitive function was recognized in male mice administered with PCP. Intraperitoneal administration of R-ketamine significantly improved the reduced cognitive function due to PCP administration (FIG. 1).

Meanwhile, intraperitoneal administration of S-ketamine did not improve the reduced cognitive function due to PCP administration (FIG. 2).

Because previous reports report that BDNF and its receptor, TrkB, are involved in the pharmaceutical action of R-ketamine (Yang C, et al. Transla. Psychiatry 2015; 5: e632. Fujita A, et al. Psychopharmacology (Berl) 2019 Aug 15; DOI: 10.1007/S00213-019-05346-5), the protein expression of BDNF, TrkB receptors, and phosphorylated TrkB in the frontal cortex and hippocampus harvested after the above behavior testing is measured. As a result, it was determined that the ratio of BDNF and p-TrkB/TrkB in the frontal cortex and the hippocampus is significantly reduced due to PCP administration but is significantly improved by R-ketamine (FIG. 3). This finding suggests that a reduction in the BDNF-TrkB system is involved in the cognitive impairment after PCP administration and that this cognitive impairment is improved by R-ketamine.

A significant reduction in cognitive function was recognized in male mice administered with PCP. Intraperitoneal administration of R-ketamine significantly improved the reduced cognitive function due to PCP administration, and intraperitoneal administration of ANA-12 suppressed the therapeutic effect of R-ketamine (FIG. 4). This finding suggests that TrkB receptors are involved in the therapeutic effect of R-ketamine on the cognitive impairment after PCP administration.

The above results clarify that intraperitoneal administration of R-ketamine at a dose of 10 mg/kg exhibits a therapeutic effect (an action of improving a cognitive impairment or a negative symptom) in mice treated with PCP serving as animal models of schizophrenia. However, intraperitoneal administration of S-ketamine at a dose of 10 mg/kg exhibited no therapeutic effect in mice treated with PCP serving as animal models of schizophrenia. It is thought that this is not due to a difference in pharmacokinetics because both ketamine isomers have the same pharmacokinetics.

Furthermore, the results of the present example confirm that S-ketamine has no effect but R-ketamine does have an effect. Because S-ketamine has a stronger affinity toward NMDA receptors, the therapeutic effect of R-ketamine in mice treated with PCP serving as animal models of schizophrenia is thought to be due to a mechanism other than an action of blocking NMDA receptors. Moreover, the results of the present example determined that the therapeutic effect of R-ketamine in mice treated with PCP serving as animal models of schizophrenia is via the BDNF-TrkB system.

Cognitive impairments are core symptoms of schizophrenia, and no improvement effect can be anticipated from current antipsychotics. From the results of the present example, it is anticipated that R-ketamine will be effective against a cognitive impairment of a neurodevelopmental disorder such as schizophrenia.

The creation of model animals of a neurodevelopmental disorder such as schizophrenia or autism spectrum disorder (models of maternal immune activation) and drug administration were specifically carried out as described below (FIG. 5). For the mice, pregnant ddY mice (8 to 10 weeks old, Japan SLC Inc., Hamamatsu, Japan) were purchased. The mice were allowed to freely ingest water and feed. Physiological saline (5 mL / kg bodyweight) or Poly (I:C) (Sigma-Aldrich: 5 mg / kg bodyweight) was intraperitoneally administered for six days, from the twelfth day of pregnancy to the seventeenth day of pregnancy, according to previous reports (Han M, et al. Sci Rep 2016; 6:36087. Matsuura A, etal.Sci Rep 2018; 8: 2158. Ma M, et al. Proc Natl Acad Sci USA 2019; 116: 7083-7088). After birth, the mice were raised normally, and at 3 weeks old, only the male mice were weaned and raised. R-ketamine (10 mg / kg bodyweight) or a medium (10 mL/kg of physiological saline) was intraperitoneally administered twice a week for four weeks, from the fourth week after birth to the eighth week after birth. This resulted in (A) a group administered with physiological saline + physiological saline, (B) a group administered with Poly (I:C) + physiological saline, and (C) a group administered with Poly (I:C) + R-ketamine. From 8 weeks old, these were raised normally. The above NORT was carried out at 10 weeks old or thereafter.

Statistical analysis was carried out by performing a one-way analysis of variance (one-way ANOVA) and subsequently performing Fisher's LSD (Fisher's LSD). Data in FIG. 5 are represented as average ± standard error (n = eight mice / group). "* p < 0.05" indicates the significant difference compared to the mouse group treated with Poly (I:C) + physiological saline.

A significant reduction in cognitive function was recognized at maturity (10 weeks old or older) in the baby mice (male) born from pregnant mice administered with Poly (I:C). Intraperitoneal administration of R-ketamine (twice a week, four weeks) suppressed a cognitive impairment at maturity in the baby mice born from pregnant mice administered with Poly (I:C) (FIG. 5).

The baby mice born from pregnant mice administered with Poly (I:C) exhibited autism-like behavioral abnormalities and prodromes of schizophrenia in childhood and exhibited autism- and schizophrenia-like behavioral abnormalities at maturity. As such, R-ketamine is thought to be useful as a preventive drug or a therapeutic drug for a neurodevelopmental disorder such as autism spectrum disorder or schizophrenia.

### [Industrial Applicability]

In the present invention, a preventive or therapeutic agent for a neurodevelopmental disorder including compound I (preferably R-ketamine), or a pharmaceutically acceptable salt thereof, as an active ingredient is useful as a novel pharmaceutical.

## Claims

1. A preventive or therapeutic agent for a neurodevelopmental disorder that includes the compound illustrated in formula (I) below, or a pharmaceutically acceptable salt thereof, as an active ingredient.
Formula (I): 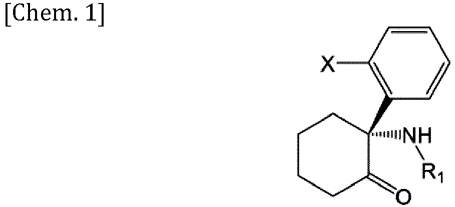 (In formula [I],
X is H, F, Cl, Br, I, an alkyl, an alkenyl, or an aryl, and
R1 is an alkyl, an alkenyl, or an aryl.)

2. The preventive or therapeutic agent for a neurodevelopmental disorder of claim 1, wherein the preventive or therapeutic agent includes R-ketamine or a pharmaceutically acceptable salt thereof.

3. The preventive or therapeutic agent for a neurodevelopmental disorder of claim 1 or 2, wherein the neurodevelopmental disorder is schizophrenia, autism spectrum disorder, a learning disorder, or attention-deficit/hyperactivity disorder.

4. The preventive agent or therapeutic agent for a neurodevelopmental disorder of any one among claims 1 to 3, wherein the neurodevelopmental disorder is a childhood neurodevelopmental disorder.

5. The preventive or therapeutic agent for a neurodevelopmental disorder of any one among claims 1 to 3, wherein the neurodevelopmental disorder is a fetal or infantile neurodevelopmental disorder.

6. The preventive agent or therapeutic agent for a neurodevelopmental disorder of any one among claims 1 to 5, wherein the neurodevelopmental disorder is related to inflammation of a mother during pregnancy.

7. The preventive or therapeutic agent for a neurodevelopmental disorder of any one among claims 1 to 6, wherein the preventive or therapeutic agent is used to prevent or treat a cognitive impairment or a negative symptom.

8. The preventive or therapeutic agent for a neurodevelopmental disorder of claim 7, wherein the cognitive impairment includes impaired attention, reduced linguistic fluidity, reduced learning and retention of linguistic information, reduced processing speed, reduced declarative memory, impaired working memory, a reduced executive function, or a combination thereof.

9. The preventive or therapeutic drug for a neurodevelopmental disorder of claim 7, wherein the negative symptom includes social withdrawal, lack of social interest, anhedonia, blunted affect, indifference or inattention toward social or cognitive input, or a combination thereof.

10. The preventive or therapeutic agent for a neurodevelopmental disorder of any one among claims 1 to 9, wherein the preventive or therapeutic agent substantially does not include the compound illustrated in formula (II) below or a pharmaceutically acceptable salt thereof.
Formula (II): 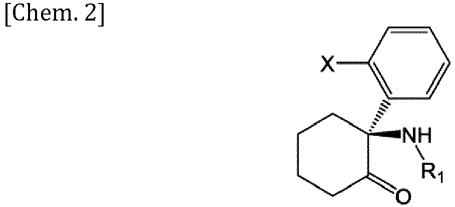 (In formula [II],
definitions of X and R1 are similar to the definitions in formula [I].)

11. The preventive or therapeutic agent for a neurodevelopmental disorder of any one among claims 1 to 10, wherein the preventive or therapeutic agent substantially does not include S-ketamine or a pharmaceutically acceptable salt thereof.

12. The preventive or therapeutic agent for a neurodevelopmental disorder of claim 11, wherein an amount of S-ketamine or a pharmaceutically acceptable salt thereof is no greater than 2% by weight.

13. The preventive or therapeutic agent for a neurodevelopmental disorder of claim 11 or 12, wherein an amount of S-ketamine or a pharmaceutically acceptable salt thereof is an amount of S-ketamine or a pharmaceutically acceptable salt thereof whereat no side effect of S-ketamine or a pharmaceutically acceptable salt thereof is exhibited.

14. The preventive or therapeutic agent for a neurodevelopmental disorder of claim 13, wherein the side effect is a psychotogenetic action.

15. A pharmaceutical composition, comprising: the preventive or therapeutic agent for a neurodevelopmental disorder of any one among claims 1 to 14.

16. The pharmaceutical composition of claim 15, wherein the pharmaceutical composition is suited for intravenous, intramuscular, subcutaneous, nasal, oral, sublingual, rectal, or percutaneous administration.

17. The pharmaceutical composition of claim 15 or 16, wherein the pharmaceutical composition is in the form of a liquid, a solution, a suspension, a powder, a tablet, a coated tablet, a capsule, a troche, a cream, a suppository, a gel, a patch, a liniment, or an aerosol.

18. The pharmaceutical composition of any one among claims 15 to 17, wherein the pharmaceutical composition includes an amount of R-ketamine that is effective in treating a neurodevelopmental disorder.

19. A preventive or therapeutic agent for a cognitive impairment in a patient that includes the compound illustrated in formula (I) below or a pharmaceutically acceptable salt thereof.
Formula (I): 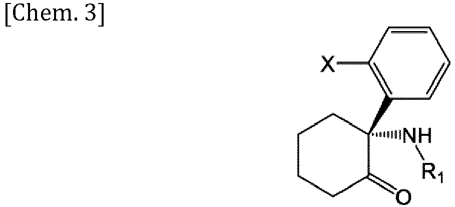 (In formula [I],
X is H, F, Cl, Br, I, an alkyl, an alkenyl, or an aryl, and
R1 is an alkyl, an alkenyl, or an aryl.)

20. The preventive or therapeutic agent for a cognitive impairment of claim 19, wherein the preventive or therapeutic agent includes R-ketamine or a pharmaceutically acceptable salt thereof.

21. The preventive or therapeutic agent for a cognitive impairment of claim 19 or 20, wherein the preventive or therapeutic agent substantially does not include the compound illustrated in formula (II) below or a pharmaceutically acceptable salt thereof. Formula (II): 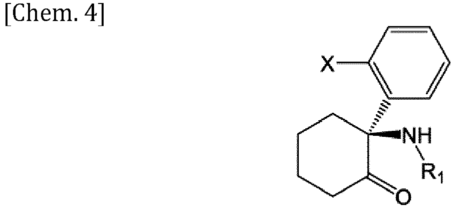 (In formula [II],
definitions of X and R1 are similar to the definitions in formula [I].)

22. The preventive or therapeutic agent for a cognitive impairment of any one among claims 19 to 21, wherein the preventive or therapeutic agent substantially does not include S-ketamine or a pharmaceutically acceptable salt thereof.

23. The preventive or therapeutic agent for a cognitive impairment of claim 22, wherein an amount of S-ketamine or a pharmaceutically acceptable salt thereof is no greater than 2% by weight.

24. The preventive or therapeutic agent for a cognitive impairment of claim 22 or 23, wherein an amount of S-ketamine or a pharmaceutically acceptable salt thereof is an amount of S-ketamine or a pharmaceutically acceptable salt thereof whereat no side effect of S-ketamine or a pharmaceutically acceptable salt thereof is exhibited.

25. The preventive or therapeutic agent for a cognitive impairment of claim 24, wherein the side effect is a psychotogenetic action.

26. The preventive or therapeutic agent for a cognitive impairment of any one among claims 19 to 25, wherein the cognitive impairment stems from a neurodevelopmental disorder.

27. The preventive agent or therapeutic agent for a cognitive impairment of claim 26, wherein the neurodevelopmental disorder is a childhood neurodevelopmental disorder.

28. The preventive or therapeutic agent for a cognitive impairment of claim 26, wherein the neurodevelopmental disorder is a fetal or infantile neurodevelopmental disorder.

29. The preventive or therapeutic agent for a cognitive impairment of any one among claims 26 to 28, wherein the neurodevelopmental disorder is schizophrenia, autism spectrum disorder, a learning disorder, or attention-deficit/hyperactivity disorder.

30. The preventive agent or therapeutic agent for a cognitive impairment of any one among claims 26 to 28, wherein the neurodevelopmental disorder is related to inflammation of a mother during pregnancy.

31. The preventive or treatment agent for a cognitive impairment of any one among claims 19 to 30, wherein the cognitive impairment includes impaired attention, reduced linguistic fluidity, reduced learning and retention of linguistic information, reduced processing speed, reduced declarative memory, impaired working memory, a reduced executive function, or a combination thereof.

32. A pharmaceutical composition, comprising: the preventive or therapeutic agent for a cognitive impairment of any one among claims 19 to 31; and a pharmaceutically acceptable carrier, diluent, or excipient.

33. The pharmaceutical composition of claim 32, wherein the pharmaceutical composition is suited for intravenous, intramuscular, subcutaneous, nasal, oral, sublingual, rectal, or percutaneous administration.

34. The pharmaceutical composition of claim 32 or 33, wherein the pharmaceutical composition is in the form of a liquid, a solution, a suspension, a powder, a tablet, a coated tablet, a capsule, a troche, a cream, a suppository, a gel, a patch, a liniment, or an aerosol.

35. The pharmaceutical composition of any one among claims 32 to 34, wherein the pharmaceutical composition includes an amount of R-ketamine that is effective in treating a cognitive impairment.
